(19) Europäisches Patentamt
European Patent Office
Office européen des brevets

(11) Publication number: 0 427 518 B1

# (12) EUROPEAN PATENT SPECIFICATION

(45) Date of publication of patent specification: **15.03.95**

(51) Int. Cl.[6]: **C07D 311/78**, A61K 31/355

(21) Application number: **90312147.3**

(22) Date of filing: **06.11.90**

(54) **NMDA-Blocking compounds, pharmaceutical compositions, their preparation and use.**

(30) Priority: **07.11.89 IL 92238**

(43) Date of publication of application:
**15.05.91 Bulletin 91/20**

(45) Publication of the grant of the patent:
**15.03.95 Bulletin 95/11**

(84) Designated Contracting States:
**AT BE CH DE DK ES FR GB GR IT LI LU NL SE**

(56) References cited:
**DE-A- 2 826 849**
**US-A- 4 133 819**
**US-A- 4 179 517**
**US-A- 4 209 520**
**US-A- 4 876 276**

(73) Proprietor: **YISSUM RESEARCH DEVELOPMENT COMPANY OF THE HEBREW UNIVERSITY OF JERUSALEM**
**46 Jabotinsky Street**
**Jerusalem, 92 182 (IL)**

Proprietor: **RAMOT UNIVERSITY, AUTHORITY FOR APPLIED RESEARCH & INDUSTRIAL DEVELOPMENT LTD.**
**Tel Aviv University,**
**32, University Street,**
**P.O. Box 39296,**
**Ramat Aviv**
**Tel Aviv 61392 (IL)**

(72) Inventor: **Kloog, Yoel**
**17 Nordau St.**
**Hertzlyia (IL)**
Inventor: **Feigenbaum, Jeffery J.**
**6250 North Talman**
**Chicago,**
**Illinois 60659 (US)**
Inventor: **Mechoulam, Raphael**
**12 Tchernichowsky St.**
**Jerusalem (IL)**
Inventor: **Sokolowsky, Mordechai**
**13 Rabina St.**
**Tel Aviv (IL)**
Inventor: **Benita, Simon**
**33 Ha'Arazim St.**
**Mevasseret Zion (IL)**

(74) Representative: **Bizley, Richard Edward et al**
**HEPWORTH LAWRENCE BRYER & BIZLEY**
**2nd Floor**
**Gate House South**
**West Gate**
**Harlow**
**Essex CM20 1JN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to pharmaceutical compositions for preventing excitatory amino acids neurotoxicity comprising as active ingredient (3S,4S)-tetrahydrocannabinol (THC) type compounds of formula (Ia), as hereunder defined.

The compositions of the present invention are particularly effective in alleviating and even preventing glutamate neurotoxicity due to acute injury to the central nervous system (CNS) such as injuries due to prolonged seizures, compromised or reduced blood supply, deprivation of glucose supply and mechanical trauma. The present compositions may also be effective in alleviating other damages to the CNS like poison-induced convulsions, considered to be associated with amino acids receptors other than that of glutamate, for example glycine.

The compositions of the present invention are also effective in the treatment of certain chronic degenerative diseases which are characterized by gradual selective neuronal loss. In this connection, the compositions of the present invention are contemplated as therapeutically effective in the treatment of the Alzheimer's disease.

The present compositions are of special value in grand mal seizures, global hypoxic ischemic insults, in hypoxia, alone or in combination with blood flow reduction (ischemia) as well as in cases of cardiac arrest and in cases of abrupt occlusion of cerebral arteries (stroke).

The compounds according to formula (Ia), as will be hereinafter described in detail, are novel compounds and are claimed as such within the scope of the present application.

The active ingredient of the pharmaceutical compositions according to the present invention is a compound of the formula:

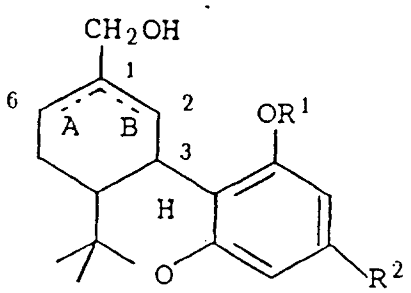

(Ia)

wherein A----B designates a 1(2) or 6(1) double bond, $R^1$ designates a lower acyl group and $R^2$ designates (A) a straight-chained or branched $C_{6-12}$ alkyl radical; or (B) a group $-O-R^3$, in which $R^3$ is a straight-chained or branched $C_{5-9}$alkyl radical or a straight-chained or branched alkyl radical substituted at the terminal carbon atom by a phenyl group, having the (3S,4S) configuration essentially free of the (3R,4R) enantiomer.

The review article "Glutamate Neurotoxicity and Diseases of the Nervous System", Neuron, 623-34 (1988) summarizes the present state of knowledge of glutamate neurotoxicity, of the various effects of such toxicity and the various diseases involved. The article describes three kinds of glutamate receptors and summarizes known competitive and noncompetitive blockers of such receptors, providing also a variety of theories on the mechanisms of such activity. According to this article, considerable efforts are being focused on bringing an N-methyl-D-aspartate (NMDA) antagonist to bear on some of the diseases associated with glutamate neurotoxicity. Since no proven effective therapy for neuronal injury, or degeneration, is yet known, and, for example, stroke alone is one of the leading causes of death in many countries, the importance of finding such NMDA antagonist is self-evident. The article states that it will be important to determine whether certain NMDA antagonists are more effective - or have fewer side-effects - than others in specific disease states.

Some of the compounds of general formula (I) as described below are disclosed in U.S. Patents Nos 4,179,517 and 4,876,276. As disclosed in said U.S. patents, these essentially pure synthetic (+)-(3S,4S)-THC derivatives and analogues are devoid of any undesired cannabimimetic psychotropic side-effects. These known compounds have been described as having analgetic, antiemetic and antiglaucoma activity.

The inventors have now found that the said known compounds, as well as some novel compounds, in addition to having said analgetic, antiemetic and anti-glaucoma activity, are also effective against the diseases and conditions mentioned above, possibly as excitatory amino acid receptor blockers, for example NMDA- or glutamate-blockers or interaction with the glycine receptor, and are effective in the alleviation and

treatment of many of the abnormal states involving said neurotransmitters toxicity.

The present invention relates to pharmaceutical compositions adapted to reduce or even prevent excitatory amino acids neurotoxicity, due to acute injury or poisoning of the CNS, such as injuries due to prolonged seizures, compromised or reduced blood supply, deprivation of glucose supply and mechanical trauma, and poisonings by, for example, strychnine, pycrotoxin or organophosphrous compounds poisonings.

The compositions of the present invention may also be effective in treating certain chronic degenerative diseases which are characterized by gradual selective neuronal loss. In this connection, the compositions of the present invention are contemplated as therapeutically effective in the treatment of Huntington's chorea, Parkinsonism disease and the Alzheimer's disease.

As stated above, the present compositions are of special value in seizures, global hypoxic ischemic insults, in hypoxia, alone or in combination with blood flow reduction (ischemia) as well as in cases of cardiac arrest and in cases of abrupt occlusion of cerebral arteries (stroke).

The present invention also relates to pharmaceutical compositions for the purposes set out above, in which the active ingredient is a compound of the general formula:

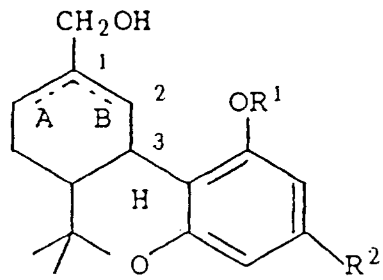

(Ia)

wherein A----B designates a 1(2) or 6(1) double bond, $R^1$ designates a lower acyl group and $R^2$ designates (A) a straight-chained or branched $C_{6-12}$ alkyl radical; or (B) a group $-O-R^3$, in which $R^3$ is a straight-chained or branched $C_{5-9}$alkyl radical or a straight-chained or branched alkyl radical substituted at the terminal carbon atom by a phenyl group, having the (3S,4S) configuration essentially free of the (3R,4R) enantiomer.

In preferred compounds $R^2$ designates 1,1-dimethylalkyl radical or 1,2-dimethylalkyl radical with a total of at least 7 carbon atoms as well as precursors of such compounds.

Particularly preferred compounds are those wherein $R^2$ is 1,1-dimethylheptyl or 1,2-dimethylheptyl.

A preferred compound,with which many of the physiological experiments have been carried out, is the compound (+)-(3S,4S)-1,1-dimethylheptyl homolog of 7-hydroxy-$\Delta^6$-tetrahydrocannabinol, designated hereafter as HU-211.

Experiments have shown that the (+)-(3S,4S) compounds of the formulae:

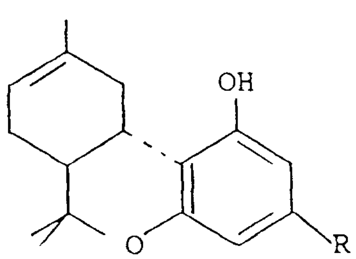

R=1,1-DMH

(II) and

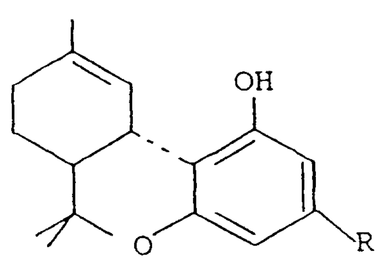

R=1,1-DMH

(IIa)

essentially free of the (-)-(3R,4R) enantiomer, wherein $R^2$ is 1,1-dimethylheptyl, have practically the same activity as that of the compound designated HU-211 (the compound of formula (II) is designated compound Vb in U.S. Patent No. 4,179,517 and the compound of formula (IIa) is compound XIb therein).

In addition it has been found that novel compounds of formula (Ia), wherein $R^1$ designates a lower acyl group also have the desired anti-glutamate or glycine-associated activity. These novel compounds may be

prepared by acetylation of compounds of formula (I) wherein R designates $CH_2OH$ and $R^1$ is hydrogen, under conditions which favour the formation of this monoester with relatively high yield. Conventional esterification, for example, acetylation of the starting compound, which has two hydroxy moieties, using acetic anhydride and pyridine yields the diacetate, the monoacetate yield not exceeding 2-3%, which is of no practical synthetic value. Inventors have now found that by conducting the esterification with an acid anhydride in the presence of potassium 2-methyl-2-butoxide, under nitrogen atmosphere, esterification on the phenyl ring hydroxy group is preferred and the desired monoester is obtained in high yield.

Among the above novel compounds the monoacetate is preferred.

It is stressed that all the compounds are of the (+)-(3S,4S) configuration, essentially free of the (-)-(3R,4R) enantiomer, the latter known to possess the undesired psychotropic side-effects. Thus, as mentioned above, compounds of the type defined by formula (Ia) are substantially devoid of "cannabis-type" CNS activity.

The novel compositions contain in addition to the active ingredient conventional pharmaceutically acceptable carriers, diluents and the like. Solid compositions for oral administration such as tablets, pills, capsules or the like may be prepared by mixing the active ingredient with conventional, pharmaceutically acceptable ingredients such as corn starch, lactose, sucrose, sorbitol, talc, stearic acid, magnesium stearate, dicalcium phosphate and gums with pharmaceutically acceptable diluents. The tablets or pills can be coated or otherwise compounded with pharmaceutically acceptable materials known in the art to provide a dosage form affording prolonged action or sustained release. Other solid compositions can be prepared as suppositories, for rectal administration. Liquid forms may be prepared for oral or administration or for injection, the term including sub-cutaneous, transdermal, intravenous, intratechal, etc administration. The liquid compositions include aqueous solutions, flavoured syrups, aqueous or oil suspensions, flavoured emulsions with edible oils, as well as elixirs and similar pharmaceutical vehicles. In addition, the compositions of the present invention may be formed as aerosols, for intra-nasal and like administration.

The active dose for humans is generally in the range of from 0.005 mg to about 50 mg per kg body weight, in regimen of 1-4 times a day. However, administration every two days may also be possible, as the drug has a rather prolonged action. The preferred range of dosage is from 1.0 mg to about 20 mg per kg body weight. However, it is evident to the man skilled in the art that dosages would be determined by the attending physician, according to the disease to be treated, method of administration, patient's age, weight, counterindications and the like.

All the compounds defined above are effective NMDA-receptor blockers and can be used as active ingredients of pharmaceutical compositions for treatment of one, or simultaneously several, of symptoms of the disorders defined above. The effective dosages are essentially similar, and the more pronounced effect is that of NMDA-receptor blocking, in addition to the known characteristics of these compounds. However, it is important to note that the compounds and compositions of the present invention exhibit good blocking activity also against convulsants which may not necessarily be NMDA-receptor blockers. For example the compositions of the present invention can prevent, or at least alleviate, poisoning caused by strychnine and NMDA.

The invention also relates to use of the compositions of the present invention for the treatment of the various pathological conditions described above. Administration of therapeutically effective amounts of the compositions of the present invention as used herein encompasses oral, parenteral, intravenous, intramuscular, sub-cutaneous, trans-dermal, intratechal, rectal and intra-nasal administration.

The enantiomers of the synthetic cannabinoid 7-hydroxy-$\Delta^6$-tetrahydrocannabionol 1,1-dimethylheptyl homolog, have been described [Mechoulam, R., et al., Tetrahedron: Asymmetry 1, 315-319 (1990); Mechoulam R et al., Experientia 44: 762-764 (1988)]. The (-)-(3R,4R) enantiomer, herein designated HU-210 (see Fig. 1), is a highly potent cannabimimetic compound (nearly 100 times more active than $\Delta$-1-tetrahydrocannabionol, the active component of hashish). The (+)-(3S,4S) enantiomer, herein designated HU-211 (Fig. 1), while known to be active as an analgesic and as an anti-emetic, is inactive as a cannabimimetic even at doses several thousand times higher than $ED_{50}$ of HU-210 [Mechoulam, R et al., Experientia 44:762-764 (1988)]. The inventors have discovered that HU-211 and the compounds of formulae (II) and (IIa), as well as the said novel monoesters which are preferred active agents of the presently claimed compositions, at about 25 mg/kg body weight, induce stereotypy, locomotor hyperactivity and tachycardia effects typically caused by NMDA-receptor antagonists. At considerably lower doses of about 2.5 mg/kg body weight, they are potent blockers of NMDA-induced tremor, seizure and lethality. Thus, a good separation has been achieved between the therapeutic effects (NMDA antagonism) and possible side effects (such as tachycardia). Binding studies show that HU-211 blocks NMDA receptors in a stereo-specific manner, and that the interaction occurs at binding sites distinct from those of other non-competitive NMDA antagonists or of glutamate and glycine. This, and the other compounds according to formula (I), may

therefore prove useful as non-psychoactive drugs that protect against NMDA-receptor-mediated neurotoxicity.

As will be shown hereafter, the pharmacological profile of HU-211, and other compounds constituting the active ingredients of the present compositions, includes the induction of stereotypy, locomotor hyperactivity and tachycardia in mice (Table 1). These properties are consistent with those of non-competitive antagonists of the NMDA sub-class of glutamate receptors, and suggest that these compounds are active NMDA-receptor antagonists. This possibility was explored by examining the activity of the compounds in protecting against the tremorogenic, convulsive and lethal effects of NMDA and NMDA agonists in mice. Such effects are counteracted by virtually all NMDA antagonists.

From the above, it will be apparent that important aspects of the invention are:-

a) the use of either:-

(i) a compound of the formula

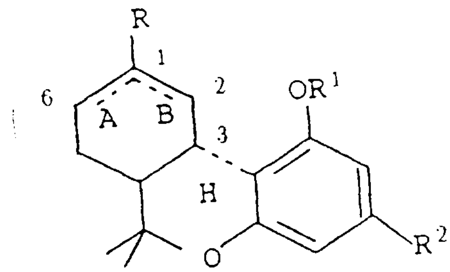

(I)

having the (3S,4S) configuration, and which is essentially free of the (3R,4R) enantiomer, wherein A----B designates a 1(2) or a 6(1) double bond, R designates $-CH_3$ or $-CH_2OH$, $R^1$ designates a hydrogen atom or a lower acyl group, and $R^2$ designates (A) a straight-chained or branched $c_{6-12}$ alkyl radical; or (B) a group $-O-R^3$, in which $R^3$ is a straight-chained or branched $C_{5-9}$alkyl radical or a straight-chained or branched alkyl radical substituted at the terminal carbon atom by a phenyl group, including those of the said compounds which are novel; or

(ii) a compound of Formula (Ia) as defined above; or both,

in the manufacture of a medicament for

(a) reducing or preventing glutamate neurotoxicity;

(b) the treatment of acute injuries to the central nervous system due to excitatory amino acid neurotoxicity, optionally for the treatment of prolonged epileptic seizures, compromised or reduced blood supply, deprivation of glucose supply, mechanical trauma, global hypoxic ischemic insults, cardiac arrest or stroke;

(c) the treatment of chronic degenerative disease associated with gradual selective neuronal loss, optionally for the treatment of Huntington's chorea, Parkinsonism or Alzheimer's disease; or

(d) the treatment of poisoning affecting the central nervous system, optionally for treating strychnine, picrotoxin or organophosphorous poisoning.

The invention will be described in detail on hand of the following Figures and Examples.

Figure 1 gives the chemical structures of HU-210 and HU211;

Figure 2 illustrates the rate of binding of [$^3$H]TCP to the NMDA-receptor channel and the rate of dissociation of [$^3$H]TCP-receptor complexes;

Figure 3 illustrates the stereoselective reduction of the rate of binding of [$^3$H]TCP to the NMDA receptor by HU-211; and

Figure 4 illustrates the non-competitive inhibition of glutamate- and glycine-induced [$^3$H]TCP binding by HU-211.

Preparatory Example

Potassium 2-methyl-butoxide (2 ml) was added to HU-211 (300 mg, 0.78 mmol) dissolved in benzene (30 ml). The solution was stirred, under $N_2$ atmosphere, for 45 min. Acetic anhydride (0.1 ml) was then added to the solution, followed by $NaHCO_3$ and water. The organic layer was dried over magnesium sulphate, filtered and evaporated. The crude reaction mixture was chromatographed on 15 g silica gel and eluted with 10%->30% ether:petrol ether solution to give:

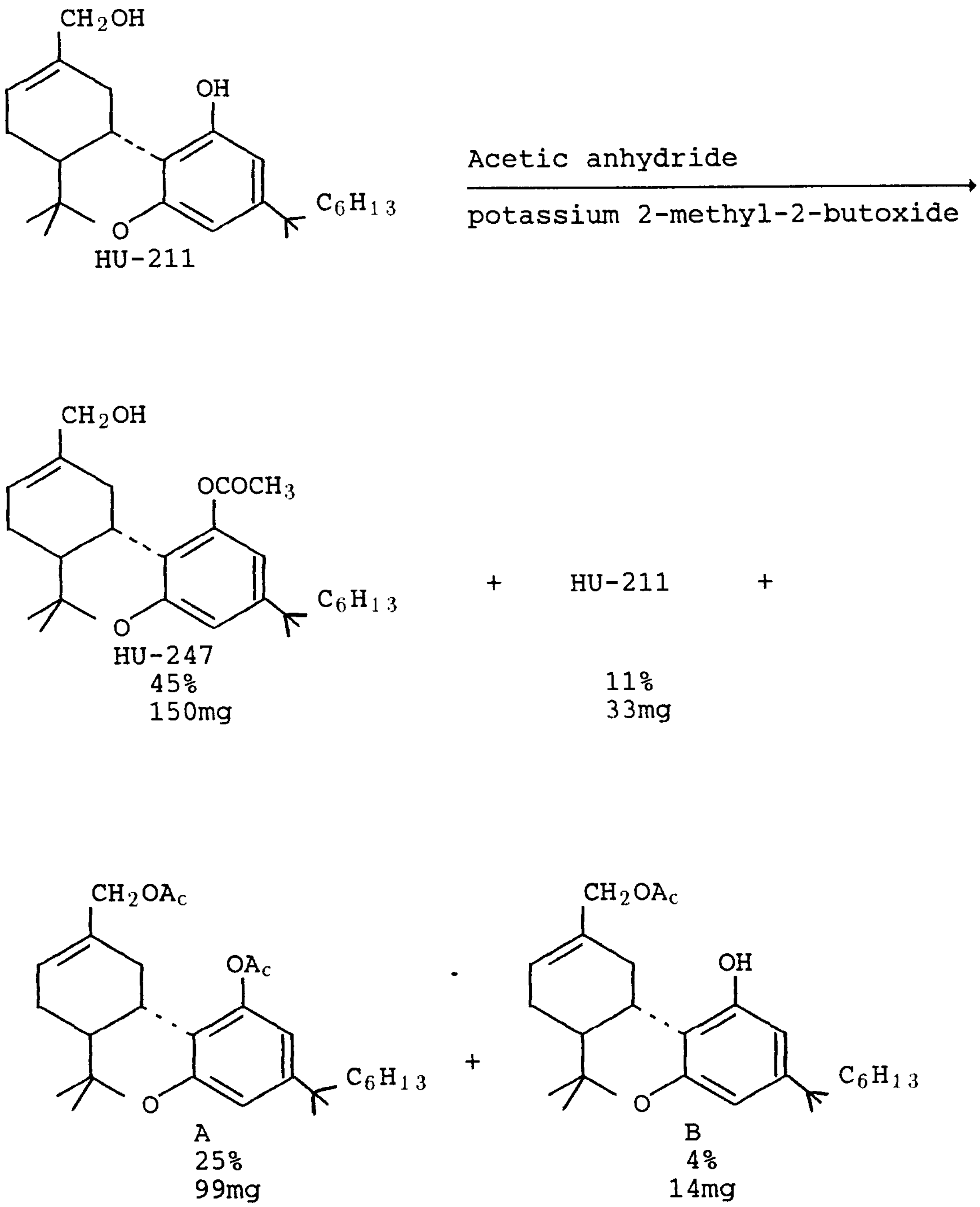


HU-211

HU-247
45%
150mg

\+ HU-211 +

11%
33mg

A
25%
99mg

B
4%
14mg

Compounds A and B were reduced with $LiAlH_4$ to recover the starting material HU-211.

Physiological Examples

Example 1

The following is a comprehensive description of the experiments conducted in order to prove the desired inhibitory activity of the compositions of the present invention against NMDA-induced tremor and convulsion as well as lethality.

Sabra mice (an Israeli heterogenous strain) were pretreated with either vehicle alone (control) or HU-211 (1.25 or 2.50 mg/kg; s.c.), followed by NMDA (200 mg/kg; s.c.). As shown in Table 2, control and HU-211 pretreated animals showed significant differences with regard to the latency of tremor, latency of seizure,

and time from NMDA injection until death. Similar experiments were performed using C-57 Black mice, in which the dose of NMDA was decreased (100 mg/kg; s.c.). Counteraction by HU-211 of the NMDA-induced effects was more pronounced in the latter strain (which is more sensitive than Sabra mice to the effects of NMDA). In the control series of both strains, which received NMDA without HU-211, all animals died in less than 10 min. following seizure onset. In contrast, two of the five Sabra mice and six of the seven C-57 Black mice pretreated with HU-211 exhibited no tremors or seizures and stayed alive for more than four days following NMDA administration (Table 2). Preliminary results indicate that the anti-NMDA effects of HU-211 in C-57 Black mice persisted for more than 24 h. This was demonstrated by the marked alleviation of NMDA-induced tremor and seizure and the continuing survival observed following the repeated administration of NMDA (100 mg/kg) one day later, in the six surviving C-57 Black mice. Taken together, these data provide compelling evidence that HU-211 is indeed an NMDA antagonist; moreover, since convulsion is a centrally-mediated phenomenon, the drug appears to exhibit a high degree of central penetration.

In order to identify the site of action of HU-211, series of binding assays were conducted, using well-washed rat brain cortical membranes and the potent non-competitive blocker of the NMDA receptor, [$^3$H]-N-[1-(2-thienyl)-cyclohexyl] piperidine ([$^3$H]TCP) (40 Ci/mmol; 1 Ci = 37 GBq; >98% pure, Israel Nuclear Research Center, Negev, Israel). With 100 $\mu$M HU-211 the equilibrium binding of 5 nM [$^3$H]TCP was not inhibited, suggesting that the behavioral effects of HU-211 are not exerted through non-competitive blocker site at the NMDA-receptor ion channel. Equilibrium binding experiments with 2-100 nM [$^3$H]TCP in the presence of 1 $\mu$M glutamate and 1 $\mu$M glycine confirmed this suggestion: HU-211 (10 $\mu$M) did not alter the maximum binding capacity of [3H]TCP (values recorded were 3.4 and 3.5 pmol/mg protein for controls and HU-211, respectively), and had no effect on the dissociation constant (Kd) for [$^3$H]TCP (27 nM for control and 26 nM for HU-211, respectively).

Kinetic experiments were then performed in order to determine whether HU-211 exerts its behavioral effects by acting directly at the glutamate- or the glycine-binding sites of the NMDA receptor. The use of this approach in previous studies, which were aimed at demonstrating the non-competitive nature of [$^3$H]-TCP and [$^3$H]MK-801 ((+)-5-methyl-10,11-dihydro-5H-dibenzol[a,d]-cyclohepten-5,10-imine maleate) binding to the NMDA receptor, showed that these compounds preferentially bind to the activated state of the receptor ion channel, and that glutamate and glycine accelerate the rates of association of non-competitive blockers to the receptor and their dissociation from it without altering their equilibrium binding. Results of the kinetic experiments with HU-211 are summarized in Figure 2. As shown, the addition of 10 $\mu$M HU-211 resulted in only a small decrease in the rate of association of [$^3$H]TCP binding to the NMDA receptor, without altering the level of its equilibrium binding; in the presence of 1 $\mu$M glutamate and 1 $\mu$M glycine, however, the addition of 10 $\mu$M HU-211 led to a marked decrease in the association rate (Figure 2A). The addition of 10 $\mu$M HU-211 also decreased the dissociation rate of [$^3$H]TCP from the NMDA receptor, both in the absence and in the presence of 1 $\mu$M glutamate and 1 $\mu$M glycine, but much more strongly in their presence.

The kinetic data thus show that HU-211 acts functionally as an NMDA antagonist in the [$^3$H]TCP binding assay. Like the competitive NMDA antagonist aminophosphovalerate (AP-5), HU-211 reduces the glutamate/glycine potentiation of [$^3$H]TCP binding. However, unlike AP-5, which strongly inhibits [$^3$H]TCP binding even in the absence of glutamate and glycine (i.e., basal binding), HU-211 appears to be a much more active blocker of [$^3$H]TCP binding when glutamate and glycine are present (i.e., induced binding). This finding, as well as the marked structural differences between AP-5 and the cannabinoid, led to considering the possibility of whether the latter does not act at the glutamate or at the glycine site. Indeed it has been found that HU-211 (10 and 5 $\mu$M) reduced the efficacy of glutamate- or glycine-induced [$^3$H]TCP binding, but did not alter their apparent affinities (not shown). Taken together, the results suggest that HU-211 exerts at least some of its behavioral effects in animals by acting at a specific site of the NMDA receptor which is distinct from the binding sites for TCP, glutamate or glycine.

Several lines of evidence suggest that the effects of HU-211 on [$^3$H]TCP binding to the NMDA receptor are indeed exerted via a specific binding site rather than through non-specific perturbations of the memebrane structure: (a) the effect on the initial rate of [$^3$H]TCP binding is dose-dependent (Figure 3), with an $IC_{50}$ value of 6-10 $\mu$M ($IC_{50}$ = dose producing 50% inhibition of glutamate- or glycine-induced binding); (b) HU-211 is a far more potent inhibitor of the induced [$^3$H]TCP binding to the NMDA receptor than its (-)enantiomer HU-210 (Figure 3), thus clearly pointing to stereospecific interaction between HU-211 and the NMDA receptor; (c) the $IC_{50}$ value for HU-211, determined under the same conditions but using phencyclidine/NMDA receptors solubilized with Na-cholate, was also 10 $\mu$M.

Recent evidence has indicated that brain damage induced by ischemia as well as by hypoglycemia is mediated, inter alia, by NMDA receptors. In light of the absence of psychotropic or other untoward side-effects seen after HU-211 administration, this drug merits serious consideration as drug of choice against

NMDA-receptor-mediated neuropathologies, including epilepsy, Huntington's chorea, and neuronal necrosis due to cerebral ischemia.

TABLE 1

**A. Effects of HU-211 (2.5 mg/kg; s.c.) on induction of stereotypy and locomotor activity in Sabra mice**

| Time (min) | 0 | 15 | 30 | 45 | 60 |
|---|---|---|---|---|---|
| | | **Stereotypy Gradient** | | | |
| Vehicle | 0.6±0.2 | 0.5±0.2 | 0.4±0.2 | 0.3±0.2 | 0.5±0.3 |
| HU-211 | 0.4±0.1 | 1.3±0.2** | 2.2±0.1** | 0.7±0.2 | 0.6±0.3 |
| | | **Locomotor Activity** | | | |
| Vehicle | 21.0±6.0 | 20.0±6.0 | 17.0±10.0 | 6.0±4.0 | 5.0±3 |
| HU-211 | 32.0±6.0 | 45.0±2.0*** | 37.0±4.04** | 28.0±13.0** | 17.0±7 |

**B. Effect of HU-211 (2.5 mg/kg; s.c.) on induction of tachycardia in Sabra mice**

| Time (min) | Heart Rate (per min) Vehicle | HU-211 | P |
|---|---|---|---|
| 0 | 170.3±2.0 | 169.2±2.5 | --- |
| 75 | 171.0±1.3 | 186.8±2.5 | <.001 |

HU-211 was dissolved in ethanol and Emulphur 620; double-distilled water was added so that ratio (by volume) in the final solution was 1:1:18. Six mice or rats were used in each experiment (including controls). Stereotypy was measured according to the stereotypy gradient SB gradient) of Feigenbaum et al., [Pharmacol. Biochem. Behav. 16:235-240 (1982)]. All animals were habituated for 45 min prior to administration of HU-211 or vehicle and tested 60 min following injection. Locomotor hyperactivity was measured as body displacement over 7-cm squares; movement from one square to the next constituted a score of 1. Heart rate was measured 0 and 75 min following HU-211 injection. Student's t-test was used for statistical analysis of locomotor activity tachycardia. Since the SB gradient consists of discrete non-continuous scores, a non-parameter test (Mann Whitney U) was used.

p<.05; **p<.01; ***p<.001. Values are means ±SEM.

TABLE 2

**A. Inhibition by HU-211 of the tremorogenic effect of NMDA**

| Mouse strain | HU-211 dosage (mg/kg) | N | Time until first tremor (min) | P |
|---|---|---|---|---|
| Sabra | 0 | 6 | 2.6 ± 0.4 | |
| | 2.5 | 5 | 8.2 ± 1.3* | <0.001 |
| C-57 Black | 0 | 6 | 3.4 ± 0.4 | |
| | 1.25 | 5 | 12.0 ± 2.6* | <0.001 |
| | 2.50 | 7 | >4 days* | |

**B. Inhibition by HU-211 of the convulsive effect of NMDA**

| Mouse strain | HU-211 dosage (mg/kg) | N | Time until first seizure (min) | P |
|---|---|---|---|---|
| Sabra | 0 | 6 | 5.2 ± 1.6 | |
| | 2.5 | 5 | 18.0 ± 7.0 | <0.001 |
| C-57 Black | 0 | 6 | 6.5 ± 0.6 | |
| | 1.25 | 5 | 13.2 ± 2.6 | <0.001 |
| | 2.50 | 6 | 4 days* | |

**C. Inhibition by HU-211 of the lethal effect of NMDA**

| Mouse strain | HU-211 dosage (mg/kg) | N | Time until death (min) | P |
|---|---|---|---|---|
| Sabra | 0 | 6 | 9.3 ± 1.4 | |
| | 2.5 | 5 | 20.2 ± 7.0 | <0.001 |
| C 57 Black | 0 | 6 | 7.7 ± 0.8 | |
| | 1.25 | 5 | 18.6 ± 4.2 | <0.001 |
| | 2.50 | 7 | >4 days* | |

Legend to Table 2

Hu-211 solution was prepared as described in Table 1. NMDA (Cambridge Research Biochemicals) was dissolved in double-distilled water. HU-211 was injected 75 min prior to NMDA administration. All injections were administered s.c. in volumes of 10 cc/kg body weight. Sabra mice were injected

with 200 mg/kg NMDA and C-57 Black mice with 100 mg/kg NMDA.

* Two out of five animals pretreated with HU-211 did not exhibit tremor or seizure and stayed alive for more than 4 days. The number relates only to the remaining animals.

** Six out of seven animals stayed alive for more than 4 days and did not exhibit tremor or seizure; statistical analysis could therefore not be performed. Re-administra -tion of NMDA (100 mg/kg) into these animals 24 h later also failed to induce tremor, seizure or death, and they stayed alive for at least 4 more days.

Values are means ± SEM.

The experiments described in Table 2 were repeated with C-57 Black mice, HU-211 replaced by the compound of formula (II) (compound Vb, U.S. Patent No. 4,179,517) and the compound of formula (IIa)-(compound XIb, ibid.). NMDA (100 mg/kg) was administered to the mice. At doses of 5 mk/kg the II and IIa compounds prevented the tremorogenic, convulsive and lethal effects in 4 out of 6 mice.

In addition, the experiments were repeated with the novel compound HU-247. HU-247 fully prevented the tremorogenic, convulsive and lethal effects of NMDA in 6 out of 7 C-57 Black mice, at doses of 2.5 mg/kg, under the same conditions described above.

The results referred to as detailed in the Figures are as follows:

Figure 2

HU-211 reduces the rate of binding of [$^3$H]TCP to the NMDA-receptor channel and the rate of dissociation of [$^3$H]TCP-receptor complexes.

A) Time course of basal (without added agonist) and induced with 1 $\mu$M L-glutamate and 1 $\mu$M glycine) [$^3$H]TCP binding to the NMDA receptor of rat brain cortical membranes. Data represent the basal binding determined in the absence (●) and in the presence (▲) of 10 $\mu$M HU-211, and the induced binding determined in the absence (o) and in the presence (△) of 10 $\mu$M HU-211. Binding of [$^3$H]TCP to well-washed control membranes was determined under the following conditions: membranes (80 $\mu$g protein) were incubated at 25°C in 200 $\mu$l HEPES buffer, pH 7.4, containing 5 nM [$^3$H]TCP (total binding) or 5 nM [$^3$H]TCP and 100 $\mu$M PCP (phencyclidine) (nonspecific binding), and the reactions were terminated at the indicated times by rapid filtration over polyethyleneimine-treated GF/C filters. The filters were counted in scintillation liquid (Hydroluma, Lumac Inc., Netherlands). HU-211 was prepared as described elsewhere [Mechoulam, R., et al., Tetrahedron:Asymmetry, 1:315-319 (1990)]. Means values (triplicates) of the specific binding of [$^3$H]TCP (total minus non-specific binding) are plotted as a function of the incubation time. The data shown are from one of three experiments that gave similar results.

(B-C) First-order plots of the basal (B) and the induced (C) dissociation of [$^3$H]TCP-receptor complexes. Data represent the basal (without added agonist) dissociation rates determined in the absence (●) and in the presence (▲) of 10 $\mu$M HU-211; the induced (with 1 $\mu$M L-glutamate and 1 $\mu$M glycine) dissociation rates were determined in the absence (o) and in the presence (△) of 10 $\mu$M HU-211. Samples were preincubated as described for Figure 2A, at 25°C with 18 nM [$^3$H]TCP for 2 h. Basal dissociation reactions were initiated by the addition of 100 $\mu$M unlabeled PCP with or without 10 $\mu$M HU-211. For the induced dissociation reactions 1 $\mu$M glutamate and 1 $\mu$M glycine were also added. Reactions were terminated either immediately (zero time) or at the indicated times. $B_{eq}$, amount of [$^3$H]TCP bound at zero time. Bt, amount of [$^3$H]TCP boud at time t. The data shown are from one of three experiments, each performed in triplicate, that gave similar results.

Figure 3

HU-211 reduces the rate of binding of [$^3$H]TCP to the NMDA receptor in a stereoselective manner.

Data represent the concentration-dependent decrease of [$^3$H]TCP binding by HU-211 (o) and by its isomer HU-210 (△). Binding of [$^3$H]TCP (5 nM) was determined as described for Figure 2A, either in the absence (basal) or in the presence (induced) of 1 µM glutamate (A) or of 1 µM glycine (B).

Reactions were terminated after 10 min. Data are expressed as induced over basal binding as a function of isomer concentrations. Basal binding was 188 fmol/mg protein. Data are from one of two experiments that gave similar results.

Figure 4

Non-competitive inhibition of glutamate- or glycine-induced [$^3$H]TCP binding by HU-211. Induced [$^3$H]-TCP binding, corresponding to the ratio of binding in the presence of glutamate or glycine to binding in their absence, was determined with 5 nM [$^3$H]TCP at 25°C for 10 min. Binding of ($^3$H)TCP to well-washed rat cortical membranes was determined in triplicate, as described previously [Kloog, Y., et al., Biochemistry, 27: 843-848 (1988)], in the absence (basal) and presence of various concentrations of L-glutamate (a) or glycine (b) with and without HU-211, as indicated. Data from one of two experiments that gave similar results.

Example 2

Protection by HU-211 against picrotoxin, strychnine and NMDA toxicity.

BALB/C mice (20 g) were subcutaneously injected with picrotoxin, strychnine and NMDA in doses of about $LD_{90}$ and subsequent clinical observations for the occurrence of convulsions and death were carried out within 20 min. The number of mice used and the mortality were assessed at the highest doses administered. Results are summarized in Table 3.

TABLE 3

| Toxin | Vehicle | Toxin Doses (mg/kg) | Time post HU-211 (min) | Doses of Hu-211 (mg/kg) | Onset time of clinical signs (min) | No.alive total | % alive |
|---|---|---|---|---|---|---|---|
| NMDA | Emulphor | 125 | - | 0 | 8 | 0/10 | 0 |
| " | " | 125 | 120 | 5 | 19 | 2/10 | 20 |
| " | Emulsion | 125 | - | 0 | - | 1/7 | 14 |
| " | " | 125 | 120 | 5 | 7 | 4/7 | 60 |
| " | " | 125 | " | 10 | 10 | 8/10 | 80 |
| Str* | Emulphor | 1 | - | 0 | - | 0/10 | 0 |
| " | " | 1 | 75 | 10 | - | 8/10 | 80 |
| Pic** | Emulsion | 4 | - | 0 | 35 | 0/6 | 0 |
| " | " | 4 | 120 | 10 | 54 | 4/8 | 50 |

*Strychnine
**Picrotoxin
Emulphor - See I, Example 3
Emulsion - See II, Example 3.

As may be realized from the results presented in Table 3, HU-211 afforded better protection against NMDA toxicity when carried in the Emulsion (Example 3, II, hereafter) than in the Emulphor (Example 3, I) preparation.

Example 3

Pharmaceutical formulations

I. Emulphor

Active agents according to the invention, for example HU-211, were dissolved in ethanol/Emulphor 620 [GAF, USA]. Bidistilled water was added. Ratio of ethanol: Emulphor:water in final solution was 1:1:18.

In general, carriers of this type may be aqueous solutions comprising pharmaceutically acceptable cosolvents, such as, for example, ethanol, polyethyleneglycol, propylenegylcol, glycerol and the like. Within this context, carriers of this type may be micellar solutions prepared with natural or synthetic ionic or non-ionic surfactants. The carriers may also be combinations of these cosolvent and micellar solutions.

II. Emulsion

In some cases, pharmaceutical preparations in the form of emulsions comprising special ingredients, detailed hereafter, may be advantageous, as has been shown in Table 3.

These emulsions are prepared from an oily phase comprising pharmaceutically acceptable triglycerides, lecitine, stabilisers, antioxidants, in which phase the active compound is solubilized, and an aqueous phase containing glycerol and emulsifiers. Co-emulsifiers may optionally be added.

Each of the oily and aqueous phases is prepared separately, the phases are combined and stirred with a magnetic stirrer, while heated to 85° C. At this temperature a coarse emulsion is achieved, using a high shear mixer. After cooling, the coarse emulsion is homogenized, to give a fine emulsion. Finally, the pH is adjusted. Preserving agents such as parabens may be added.

Preferred triglycerides are medium- and long-chain triglycerides. Deoxycholate is a preferred stabiliser. α-Tocopherol is a preferred anti-oxidant. Pluronic F-68 is a preferred emulsifier.

Thus, a general example of a desired formulation of an "Emulsion", referred to in Table 3 may be: triglycerides (from 1.0% to 30%, preferably 10%), lecitine (from 0.10% to 5%, preferably 1%), antioxidant, (% as required, according to specific anti-oxidant used), stabiliser (from 0.05% to 5%, preferably 1%), glycerol (% as required for isotonicity), emulsifier (from 0.20% to 10%, preferably 2%), active compound (from 0.05% to 5%) and water up to 100% (all %w/w).

Preserving agents such as parabens may be added (0.01-0.1%).

A typical formulation (%w/w): HU-211 (1.0) stabilized in oily phase (20.0), purified fractionated egg yolk phospholipids (1.2), Pluronic F-68 (2.0), glycerol (2.25), α-tocopherol (0.02), methyl-, butyl-p-hydroxybenzoic acid esters ((0.2) and (0.075), respectively) and bidistilled water (up to 100).

**Claims**

**1.** A compound of the formula:

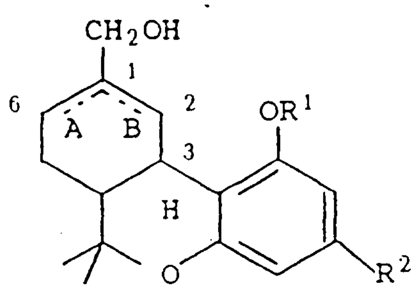

(Ia)

wherein A----B designates a 1(2) or 6(1) double bond, $R^1$ designates a lower acyl group and $R^2$ designates (A) a straight-chained or branched $C_{6-12}$ alkyl radical; or (B) a group -O-$R^3$, in which $R^3$ is a straight-chained or branched $C_{5-9}$alkyl radical or a straight-chained or branched alkyl radical substituted at the terminal carbon atom by a phenyl group, having the (3S,4S) configuration essentially free of the

(3R,4R) enantiomer.

2. A compound according to claim 1 wherein $R^1$ is acetyl and $R^2$ is 1,1-dimethylheptyl or 1,2-dimethyl heptyl.

3. A process for the preparation of a compound of formula (Ia) as defined in claim 1 comprising reacting a compound of the formula:

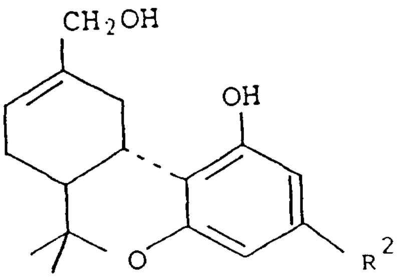

with potassium 2-methyl-2-butoxide, under $N_2$ atmosphere, adding acid anhydride, upon completion of the reaction adding sodium bicarbonate and water, drying the organic layer over magnesium sulphate, filtering and evaporating this organic layer and separating the desired monoester by silica gel chromatography.

4. A process according to claim 3 wherein $R^2$ is 1,1-dimethylheptyl or 1,2-dimethylheptyl and said acid anhydride is acetic anhydride.

5. A pharmaceutical NMDA-blocking composition reducing and preventing glutamate neurotoxicity, containing as active ingredient an effective NMDA-blocking quantity of a compound of the formula:

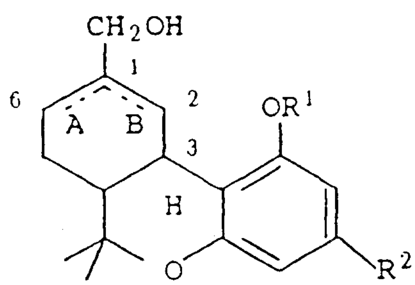

(Ia)

wherein A----B designates a 1(2) or 6(1) double bond, $R^1$ designates a lower acyl group and $R^2$ designates (A) a straight-chained or branched $C_{6-12}$ alkyl radical; or (B) a group $-O-R^3$, in which $R^3$ is a straight-chained or branched $C_{5-9}$alkyl radical or a straight-chained or branched alkyl radical substituted at the terminal carbon atom by a phenyl group, having the (3S,4S) configuration essentially free of the (3R,4R) enantiomer, with a pharmaceutically acceptable carrier or diluent, optionally wherein said active ingredient is a compound according to either claim 2 or claim 3 and/or wherein said composition is in unit dosage form.

6. A composition according to claim 5, where the active ingredient is a compound of Formula (Ia) wherein $R^2$ is 1,1-dimethylheptyl or 1,2-dimethylheptyl in the essentially pure (3S,4S) form.

7. A pharmaceutical composition according to claim 5 or claim 6 wherein said carrier is an aqueous cosolvent solution comprising a pharmaceutically acceptable cosolvent, or a micellar solution prepared with natural or synthetic ionic or non-ionic sufactants, or a combination of such cosolvent and micellar solutions, optionally wherein said carrier is an ethanol/Emulphor 620/water solution; or wherein said

carrier is an emulsion comprising conventional pharmaceutically acceptable emulsifiers, stabilisers and optionally co-emulsifiers, and water, if desired further comprising Pluronic F-68.

**8.** A pharmaceutical composition according to any one of claims 5 to 7 for:
(a) the treatment of acute injuries to the central nervous system due to excitatory amino acid neurotoxicity, optionally for the treatment of prolonged epileptic seizures, compromised or reduced blood supply, deprivation of glucose supply, mechanical trauma, global hypoxic ischemic insults, cardiac arrest or stroke;
(b) the treatment of chronic degenerative disease associated with gradual selective neuronal loss, optionally for the treatment of Huntington's chorea, Parkinsonism or Alzheimer's disease; or
(c) the treatment of poisoning affecting the central nervous system, optionally for treating strychnine, picrotoxin or organophosphorous poisoning.

**9.** The use of either (i) a compound of the Formula

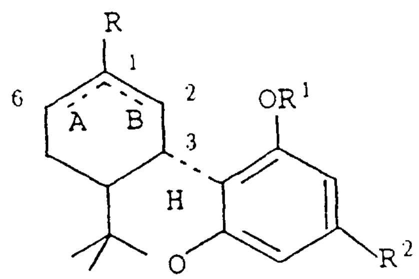

(I)

having the (3S,4S) configuration, and which is essentially free of the (3R,4R) enantiomer, wherein A----B designates a 1(2) or a 6(1) double bond, R designates $-CH_3$ or $-CH_2OH$, $R^1$ designates a hydrogen atom or a lower acyl group, and $R^2$ designates (A) a straight-chained or branched $c_{6-12}$ alkyl radical; or (B) a group $-O-R^3$, in which $R^3$ is a straight-chained or branched $C_{5-9}$alkyl radical or a straight-chained or branched alkyl radical substituted at the terminal carbon atom by a phenyl group, or (ii) a compound of the Formula (Ia) as defined in claim 1, in the manufacture of a medicament for
(a) reducing or preventing glutamate neurotoxicity;
(b) the treatment of acute injuries to the central nervous system due to excitatory amino acid neurotoxicity, optionally for the treatment of prolonged epileptic seizures, compromised or reduced blood supply, deprivation of glucose supply, mechanical trauma, global hypoxic ischemic insults, cardiac arrest or stroke;
(c) the treatment of chronic degenerative disease associated with gradual selective neuronal loss, optionally for the treatment of Huntington's chorea, Parkinsonism or Alzheimer's disease; or
(d) the treatment of poisoning affecting the central nervous system, optionally for treating strychnine, picrotoxin or organophosphorous poisoning.

**10.** The use of a compound of Formula (Ia) wherein $R^1$ is a hydrogen atom and $R^2$ is 1,1-dimethylheptyl or 1,2-dimethylheptyl in the essentially pure (3S,4S) form wherein the active compound is (3S,4S)-(+)-1,1,dimethylheptyl homolog of 7-hydroxy-delta$^{-6-}$ tetrahydrocannabinol in the manufacture of a medicament for reducing and preventing glutamate neurotoxicity.

**Patentansprüche**

1. Verbindung der Formel

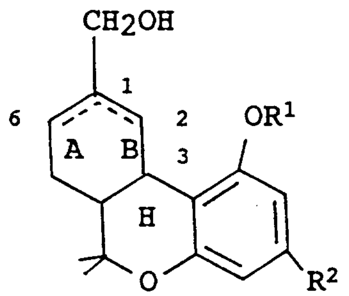

(Ia)

worin A----B eine 1(2) oder 6(1) Doppelbindung bezeichnet, $R^1$ eine niedrige Acylgruppe bezeichnet und $R^2$ (A) einen geradkettigen oder verzweigten $C_{6-12}$ Alkylrest; oder (B) eine -O-$R^3$-Gruppe bezeichnet, in welcher $R^3$ ein geradkettiger oder verzweigter $C_{5-9}$ Alkylrest oder ein geradkettiger oder verzweigter Alkylrest ist, welcher an dem terminalen Kohlenstoffatom durch eine Phenylgruppe substituiert ist, welcher Rest die im wesentlichen von dem (3R,4R) Enantiomeren freie (3S,4S) Konfiguration aufweist.

2. Verbindung nach Anspruch 1, worin $R^1$ Acetyl ist und $R^2$ 1,1-Dimethylheptyl oder 1,2-Dimethylheptyl ist.

3. Verfahren zur Herstellung einer Verbindung der wie in Anspruch 1 definierten Formel (Ia), umfassend das Umsetzen einer Verbindung der Formel:

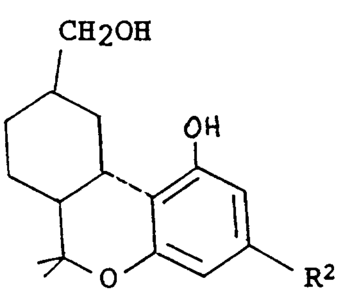

mit Kalium 2-Methyl-2-butoxid unter einer $N_2$-Atmosphäre Zusetzen von Säureanhydrid, nach Vervollständigung der Reaktion Zusetzen von Natriumbicarbonat und Wasser, Trocknen der organischen Schicht über Magnesiumsulfat, Filtrieren und Verdampfen dieser organischen Schicht und Abtrennen des gewünschten Monoesters durch Kieselgelchromatographie.

4. Verfahren nach Anspruch 3, worin $R^2$ 1,1-Dimethylheptyl oder 1,2-Dimethylheptyl ist und das Säureanhydrid Essigsäureanhydrid ist.

5. Pharmazeutische NMDA-blockierende Zusammensetzung, welche die Glutamat-Neurotoxizität reduziert und vorbeugt, enthaltend als wirksamen Bestandteil eine effiziente, NMDA-blockierende Menge einer Verbindung der Formel:

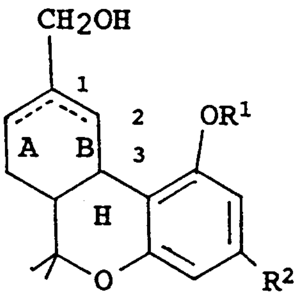

(Ia)

worin A----B eine 1(2) oder 6(1) Doppelbindung bezeichnet, $R^1$ eine niedrige Acylgruppe bezeichnet und $R^2$ (A) einen geradkettigen oder verzweigten $C_{6-12}$ Alkylrest; oder (B) eine -O-$R^3$-Gruppe bezeichnet, in welcher $R^3$ ein geradkettiger oder verzweigter $C_{5-9}$ Alkylrest oder ein geradkettiger oder verzweigter Alkylrest ist, welcher an dem terminalen Kohlenstoffatom durch eine Phenylgruppe substituiert ist, welcher Rest die im wesentlichen von dem (3R,4R) Enantiomeren freie (3S,4S) Konfiguration aufweist, mit einem pharmazeutisch verträglichen Träger oder Verdünnungsmittel, worin gegebenenfalls der wirksame Bestandteil eine Verbindung nach einem der Ansprüche 2 oder 3 ist und/oder worin die Zusammensetzung in Form einer Einheitsdosierung vorliegt.

**6.** Zusammensetzung nach Anspruch 5, worin der wirksame Bestandteil eine Verbindung der Formel (Ia) ist, worin $R^2$ 1,1-Dimethylheptyl oder 1,2-Dimethylheptyl in der im wesentlichen reinen (3S,4S) Form ist.

**7.** Pharmazeutische Zusammensetzung nach Anspruch 5 oder 6, worin der Träger ein wässriges Colösungsmittel, umfassend ein pharmazeutisch verträgliches Colösungsmittel, oder eine micellare Lösung, welche mit natürlichen oder synthetischen ionischen oder nicht-ionischen oberflächenaktiven Substanzen hergestellt ist, oder eine Kombination aus einem derartigen Colösungsmittel und micellaren Lösungen ist, wobei gegebenenfalls dieser Träger eine Ethanol/Emulphor 620/Wasser Lösung ist; oder worin dieser Träger eine Emulsion, umfassend übliche pharmazeutisch verträgliche Emulgatoren, Stabilisierungsmittel und gegebenenfalls Co-Emulgatoren und Wasser und falls gewünscht weiters Pluronic F-68 ist.

**8.** Pharmazeutische Zusammensetzung nach einem der Ansprüche 5 bis 7 für:

(a) die Behandlung von akuten Schädigungen des Zentralnervensystems aufgrund von exzitatorischer Aminosäure-Neurotoxizität, gegebenenfalls für die Behandlung von langdauernden epileptischen Anfällen, beeinträchtigter oder reduzierter Blutversorgung, Deprivationen der Glukoseversorgung, mechnischem Trauma, globalen hypoxischen ischämischen Anfällen, Herzstillstand oder Schlaganfall;

(b) die Behandlung von chronischen degenerativen Erkrankungen, welche mit stufenweise fortschreitenden selektiven neuronalen Verlust verbunden sind, gegebenenfalls für die Behandlung von Huntington-Chorea, Parkinsonismus oder Alzheimer-Krankheit; oder

(c) die Behandlung von Vergiftungen, welche das Zentralnervensystem beeinflussen, gegebenenfalls für die Behandlung von Vergiftungen mit Strychnin, Picrotoxin oder Organophosphorverbindungen.

**9.** Verwendung von entweder (i) einer Verbindung der Formel

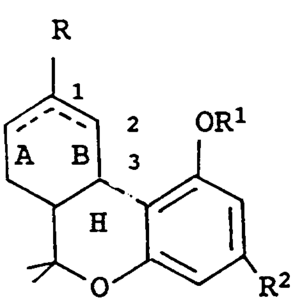

(Ia)

welche die (3S,4S) Konfiguration aufweist, und welche im wesentlichen frei von dem (3R,4R) Enantiomeren ist, worin A----B eine 1(2) oder 6(1) Doppelbindung bezeichnet, R $-CH_3$ oder $-CH_2OH$ bezeichnet, $R^1$ ein Wasserstoffatom oder eine niedrige Acylgruppe bezeichnet und $R^2$ (A) einen geradkettigen oder verzweigten $C_{6-12}$ Alkylrest; oder (B) eine $-O-R^3$-Gruppe bezeichnet, in welcher $R^3$ ein geradkettiger oder verzweigter $C_{5-9}$ Alkylrest oder ein geradkettiger oder verzweigter Alkylrest, welcher an dem terminalen Kohlenstoffatom durch eine Phenylgruppe substituiert ist, bedeutet, oder (ii) eine Verbindung der Formel (Ia), welche wie in Anspruch 1 definiert ist, bei der Herstellung eines Medikamentes für

(a) die Reduktion oder Vorbeugung von Glutamat-Neurotoxizität;

(b) die Behandlung von akuten Schädigungen des Zentralnervensystems aufgrund von exzitatorischer Aminosäure-Neurotoxizität, gegebenenfalls für die Behandlung von langdauernden epileptischen Anfällen, beeinträchtigter oder reduzierter Blutversorgung, Deprivationen der Glukoseversorgung, mechnischem Trauma, globalen hypoxischen ischämischen Anfällen, Herzstillstand oder Schlaganfall;

(c) die Behandlung von chronischen degenerativen Erkrankungen, welche mit stufenweise fortschreitendem selektiven neuronalen Verlust verbunden sind, gegebenenfalls für die Behandlung von Huntington-Chorea, Parkinsonismus oder Alzheimer-Krankheit; oder

(d) die Behandlung von Vergiftungen, welche das Zentralnervensystem beeinflussen, gegebenenfalls für die Behandlung von Vergiftungen mit Strychnin, Picrotoxin oder Organophosphorverbindungen.

**10.** Verwendung einer Verbindung der Formel (Ia), worin $R^1$ ein Wasserstoffatom und $R^2$ 1,1-Dimethylheptyl oder 1,2-Dimethylheptyl in der im wesentlichen reinen (3S,4S) Form ist, worin die wirksame Verbindung das (3S,4S)-(+)-1,1-Dimethylheptyl-Homologe von 7-Hydroxy-delta$^{-6-}$tetrahydrocannabinol ist, bei der Herstellung eines Medikamentes zur Reduktion oder Vorbeugung von Glutamat-Neurotoxizität.

## Revendications

**1.** Composé de formule :

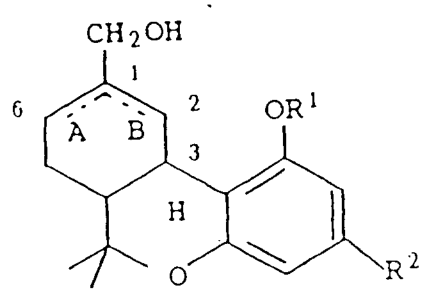

(Ia)

dans laquelle A----B représente une double liaison 1(2) ou 6(1), $R^1$ représente un groupe acyle inférieur et $R^2$ représente (A) un groupe alkyle en $C_{6-12}$ à chaîne droite ou ramifiée; ou (B) un groupe $-O-R^3$, dans lequel $R^3$ est un groupe alkyle en $C_{5-9}$ à chaîne droite ou ramifiée ou un groupe alkyle à chaîne droite ou ramifiée substitué sur l'atome de carbone terminal par un groupe phényle, ce composé possédant la configuration (3S,4S), essentiellement exempt de l'énantiomère (3R,4R).

**2.** Composé selon la revendication 1, dans lequel $R^1$ est le groupe acétyle et $R^2$ est le groupe 1,1-diméthylheptyle ou le groupe 1,2-diméthylheptyle.

**3.** Procédé de préparation d'un composé de formule (Ia) tel que défini dans la revendication 1, comprenant la mise en réaction d'un composé de formule:

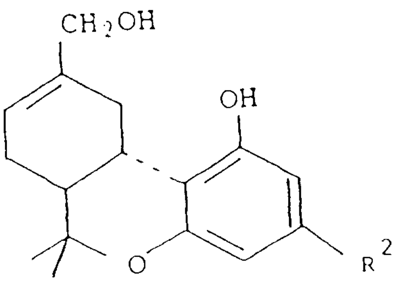

avec du 2-méthyl-2-butylate de potassium sous une atmosphère d'azote, l'ajout d'un anhydride d'acide, et après réaction complète, l'ajout de bicarbonate de sodium et d'eau, le séchage de la phase organique sur du sulfate de magnésium, le filtrage et l'évaporation de cette phase organique, et la séparation du monoester souhaité par chromatographie sur gel de silice.

**4.** Procédé selon la revendication 3, dans lequel $R^2$ est le groupe 1,1-diméthylheptyle ou le groupe 1,2-diméthylheptyle et ledit anhydride d'acide est l'anhydride acétique.

**5.** Composition pharmaceutique pour le blocage du NMDA permettant la réduction et la prévention de la neurotoxicité du glutamate, contenant en tant qu'ingrédient actif une quantité efficace pour le blocage du NMDA d'un composé de formule

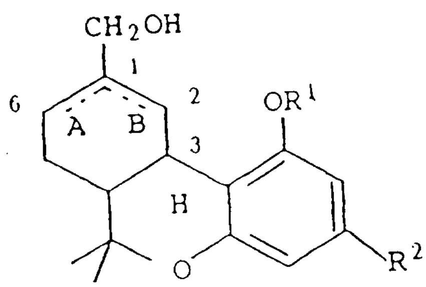

(Ia)

dans laquelle A----B représente une double liaison 1(2) ou 6(1), $R^1$ représente un groupe acyle inférieur et $R^2$ représente (A) un groupe alkyle en $C_{6-12}$ à chaîne droite ou ramifiée; ou (B) un groupe -O-$R^3$, dans lequel $R^3$ est un groupe alkyle en $C_{5-9}$ à chaîne droite ou ramifiée ou un groupe alkyle à chaîne droite ou ramifiée substitué sur l'atome de carbone terminal par un groupe phényle, ce composé possédant la configuration (3S,4S), essentiellement exempt de l'énantiomère (3R,4R),
avec un véhicule ou un diluant pharmaceutiquement acceptable, éventuellement dans laquelle ledit ingrédient actif est un composé selon la revendication 2 ou la revendication 3 et/ou ladite composition est sous la forme de dose unitaire.

**6.** Composition selon la revendication 5, où l'ingrédient actif est un composé de formule (Ia) dans laquelle $R^2$ est le groupe 1,1-diméthylheptyle ou le groupe 1,2-diméthylheptyle, sous la forme (3S,4S) essentiellement pure.

**7.** Composition pharmaceutique selon la revendication 5 ou la revendication 6, dans laquelle ledit véhicule est une solution aqueuse d'un cosolvant comprenant un cosolvant pharmaceutiquement acceptable, ou une solution micellaire préparée avec des tensioactifs ioniques ou non-ioniques naturels ou synthétiques, ou une combinaison de telles solutions de cosolvant et micellaire, éventuellement dans laquelle ledit véhicule est une solution éthanol/Emulphor 620/eau; ou dans laquelle ledit véhicule est une émulsion comprenant des émulsifiants, stabilisants, et éventuellement des co-émulsifiants, habituels pharmaceutiquement acceptables, et de l'eau, comprenant en outre si on le désire du Pluronic F-68.

**8.** Composition pharmaceutique selon l'une quelconque des revendications 5 à 7 pour :

(a) le traitement de lésions aiguës du système nerveux central dues à la neurotoxicité d'un amino acide excitateur, éventuellement pour le traitement de crises épileptiques prolongées, de réductions ou de risques de pertes d'apport sanguin, de carences en apport de glucose, de traumatismes mécaniques, de troubles globaux ischémiques et hypoxiques, d'arrêt cardiaque ou de crise cardiaque;
(b) le traitement de maladies dégénératives chroniques associées à une perte neuronale sélective graduelle, éventuellement pour le traitement de la chorée de Huntington, de la maladie de Parkinson ou d'Alhzeimer; ou
(c) le traitement d'empoisonnements affectant le système nerveux central, éventuellement pour le traitement d'empoisonnement par la strychnine, la picrotoxine ou des composés organophosphorés.

**9.** Utilisation soit (i) d'un composé de formule

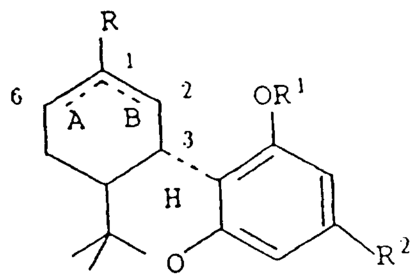

(I)

possédant la configuration (3S,4S), et qui est essentiellement exempt de l'énantiomère (3R,4R), formule dans laquelle A----B représente une double liaison 1(2) ou une double liaison 6(1); R représente un groupe $-CH_3$ ou $-CH_2OH$, $R^1$ représente un atome d'hydrogène ou un groupe acyle inférieur, et $R^2$ représente (A) un groupe alkyle en $C_{6-12}$ à chaîne droite ou ramifiée; ou (B) un groupe $-OR^3$, dans lequel $R^3$ est un groupe alkyle en $C_{5-9}$ à chaîne droite ou ramifiée ou un groupe alkyle à chaîne droite ou ramifiée substitué sur l'atome de carbone terminal par un groupe phényle, soit (ii) d'un composé de formule (Ia) tel que défini dans la revendication 1, dans la préparation d'un médicament pour
(a) la réduction ou la prévention de la neurotoxicité du glutamate;
(b) le traitement de lésions aiguës du système nerveux central dues à la neurotoxicité d'un amino acide excitateur, éventuellement pour le traitement de crises épileptiques prolongées, de réductions ou de risques de pertes d'apport sanguin, de carences en apport de glucose, de traumatismes mécaniques, de troubles globaux ischémiques et hypoxiques, d'arrêt cardiaque ou de crise cardiaque;
(c) le traitement de maladies dégénératives chroniques associées à une perte neuronale sélective graduelle, éventuellement pour le traitement de la chorée de Huntington, de la maladie de Parkinson ou d'Alhzeimer; ou
(d) le traitement d'empoisonnements affectant le système nerveux central, éventuellement pour le traitement d'empoisonnement par la strychnine, la picrotoxine ou des composés organophosphorés.

**10.** Utilisation d'un composé de formule (Ia) dans laquelle $R^1$ est un atome d'hydrogène et $R^2$ est le groupe 1,1-diméthylheptyle ou le groupe 1,2-diméthylheptyle, sous une forme (3S,4S) essentiellement pure, utilisation dans laquelle le composé actif est le composé (3S,4S)-(+)-1,1-diméthylheptyle homologue du 7-hydroxy-delta$^6$-tétrahydrocannabinol, dans la préparation d'un médicament pour la réduction et la prévention de la neurotoxicité du glutamate.

Figure 1

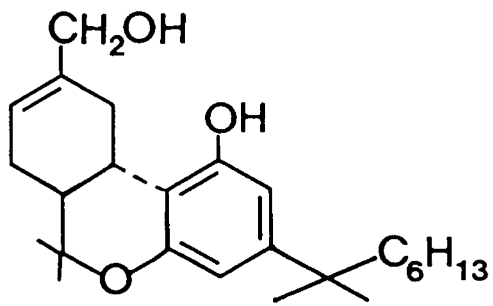

HU — 211

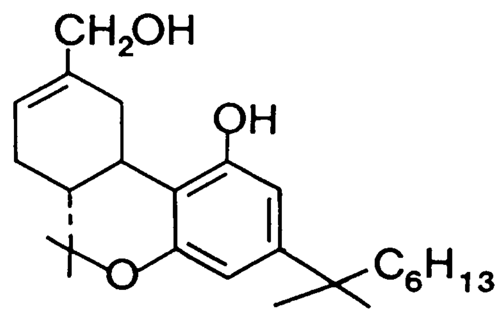

HU — 210

Figure 2

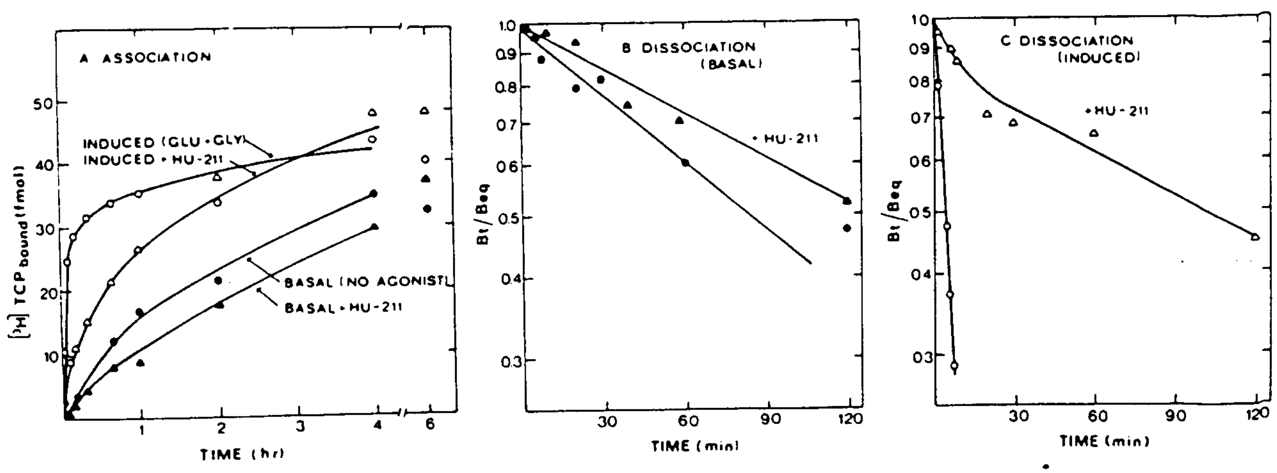

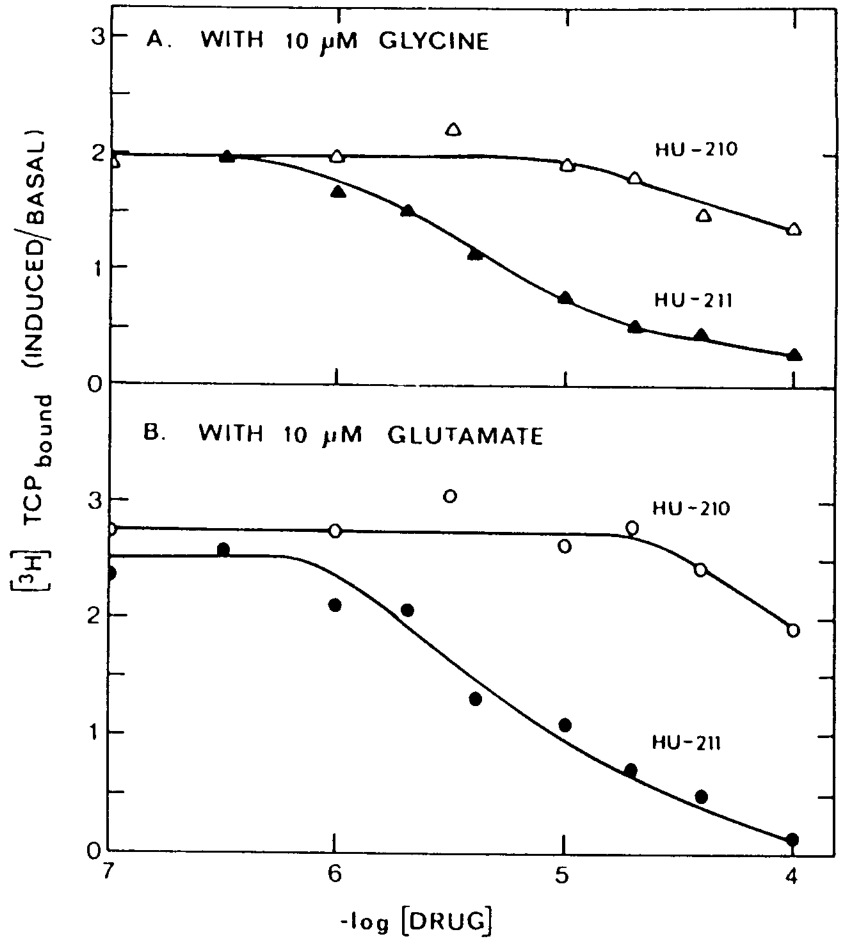


Figure 3

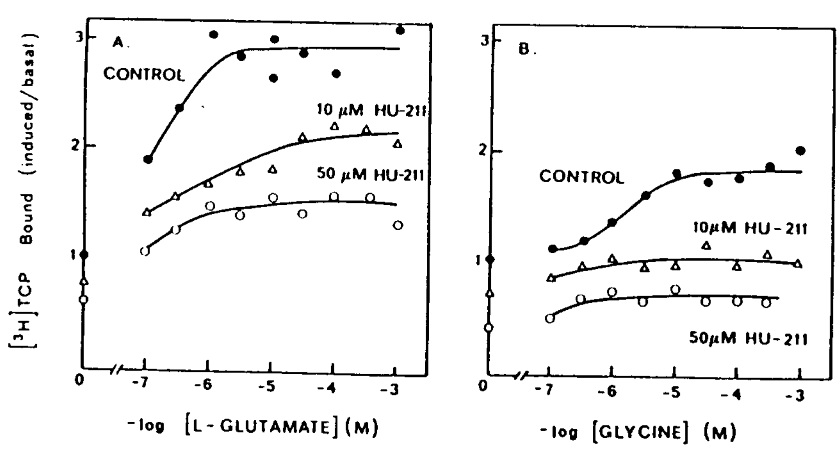


Figure 4